# EUROPÄISCHE PATENTSCHRIFT

(11) **EP 1 264 602 B1**
(45) Veröffentlichungstag und Bekanntmachung des Hinweises auf die Patenterteilung: **05.07.2006**
(21) Anmeldenummer: 02010383.4
(22) Anmeldetag: 08.05.2002
(51) Int. Cl.: A61K 47/02, A61K 8/25

(54) **Pharmazeutische Zubereitung enthaltend pyrogenes Siliziumdioxid von hoher Stampfdichte**
Pharmaceutical composition comprising high tapped density fumed silica
Composition pharmaceutique contenant une silice pyrogénique à haute densité tassée

(30) Priorität: 30.05.2001 DE 10126163
(43) Veröffentlichungstag der Anmeldung: 11.12.2002
(73) Patentinhaber: Degussa AG, 40474 Düsseldorf (DE)
(72) Erfinder: Hasenzahl, Steffen, Dr., 63454 Hanau (DE); Drechsler, Margarete, 63571 Gelnhausen (DE); Hirschhäuser, Michael, 61250 Usingen-Eschbach (DE)

(56) Entgegenhaltungen:
- EP-A- 1 172 095
- EP-A- 1 174 122
- US-A- 6 051 253
- US-B1- 6 217 909
- DATABASE AEROSIL HOMEPAGE [Online] Degussa AG; retrieved from WWW.AEROSIL.COM XP002216088

## Beschreibung

Gegenstand der Erfindung ist die Verwendung von pyrogenem Siliziumdioxid als Hilfsstoff zur Herstellung von Kapseln oder Tabletten, **dadurch gekennzeichnet, dass** das pyrogene Siliziumdioxid eine Stampfdichte von 100 bis 200 g/l sowie eine Spezifische Oberfläche zwischen 90 und 250 m²/g aufweist.

Bei einem Arzneimittel lassen sich in der Regel zwei funktional verschiedene Stoffgruppen unterscheiden, nämlich Wirkstoffe und Hilfsstoffe.

Wirkstoffe sind durch ihre spezifische pharmakologische Wirkung gekennzeichnet. Sie stellen den wirksamen Bestandteil eines Arzneimittels dar. Als solche sind sie auf der Verpackung und auf dem Beipackzettel auch quantitativ ausgewiesen.

Hilfsstoffe dagegen haben keine pharmakologische Wirkung. Sie werden benötigt, um für den Wirkstoff eine geeignete Darreichungsform, nämlich das Arzneimittel, herstellen zu können. In der Regel enthält das Arzneimittel mehrere Hilfsstoffe mit unterschiedlichen Funktionen. Zum Beispiel dienen Hilfsstoffe als Füllstoffe, Bindemittel, Sprengmittel, Gleitmittel, Schmiermittel oder Formtrennmittel.

Bei der Entwicklung von stabilen, gut handhabbaren und wirksamen Arzneimitteln aus Wirkstoff(en) und Hilfsstoffen kann auf eine Vielzahl von Hilfsstoffen zurückgegriffen werden.

Häufig wird hochdisperses, pyrogenes Siliziumdioxid, zum Beispiel Aerosil® , in pharmazeutischen und kosmetischen Zubereitungen eingesetzt. In festen Produktformen kann es als Fließregulierungs-, Adsorptions- und Trocknungsmittel verwendet werden, in flüssigen und halbflüssigen Produktformen als Suspensionsstabilisator, Gerüst- und Gelbildner. Weiterhin kann es eingesetzt werden, um die mechanische Stabilität und die Zerfallsgeschwindigkeit von Tabletten zu erhöhen. Außerdem kann es die Wirkstoffverteilung verbessern. Hochdisperses pyrogenes Silicumdioxid kann aber auch als Wirkstoff fungieren, zum Beispiel zur Behandlung von Gastritis, Enteritis oder Hautverbrennungen (Schriftenreihe Pigmente Nr. 49, "AEROSIL in Pharmaceuticals and Cosmetics, Degussa AG).

Nachteilig beim Arbeiten mit hochdispersem Siliziumdioxid ist besonders die Staubentwicklung, da bei der Herstellung pharmazeutischer und kosmetischer Produkte höchste Reinheitsanforderungen erfüllt werden müssen.

Ein weiterer Nachteil ist die geringe Schütt- und Stampfdichte von üblicherweise verwendetem hochdispersem Siliziumdioxid, wodurch ein beträchtlicher zusätzlicher Arbeits- und Zeitaufwand bei der Herstellung pharmazeutischer und kosmetischer Zubereitungen verursacht wird.

Wünschenswert bei der Verwendung von hochdispersem Siliziumdioxid in pharmazeutischen und kosmetischen Zubereitungen wäre ferner eine verbesserte Fließfähigkeit damit hergestellter Mischungen, um zum Beispiel bei der Herstellung von Tabletten und Kapseln eine höhere Dosiergenauigkeit erreichen zu können. Dadurch wäre zum einen eine geringere Varianz von Tabletten- und Kapselgewichten zu erzielen und zum anderen die Wirtschaftlichkeit von Prozessen, die zu diesen Darreichungsformen führen zu verbessern.

Aufgabe der vorliegenden Erfindung ist es pharmazeutische Zubereitungen bereitzustellen, die die Nachteile des Standes der Technik vermeiden.

Die Aufgabe wird dadurch gelöst, dass man pharmazeutische und kosmetische Zubereitungen herstellt und einsetzt, die pyrogenes Siliziumdioxid als Hilfsstoff enthalten, welches eine Stampfdichte von 100 bis 200 g/l, bestimmt nach DIN 55943, sowie eine spezifische Oberfläche zwischen 90 und 250 m²/g aufweist.

Dieses Ergebnis ist überraschend, da man nicht davon ausgehen konnte, dass die Eigenschaften, wie zum Beispiel Staubentwicklung, Fließfähigkeit oder mechanische Stabilität, der pharmazeutischen Zubereitungen von der Stampfdichte des pyrogenen Siliziumdioxides beeinflusst werden.

Es wurde gefunden, dass beim Arbeiten mit dem erfindungsgemäßen Verfahren nur eine geringe Staubentwicklung auftritt und die Fließfähigkeit der Zubereitungen deutlich höher ist als bei solchen nach dem Stand der Technik. Daneben wird die mechanische Stabilität von Tabletten verbessert und das Kapselgewicht erhöht.

Es hat sich gezeigt, dass es besonders günstig ist die Stampfdichte des Siliziumdioxides zwischen 100 und 200 g/l zu wählen. Besonders bevorzugt ist ein hochdisperses Siliciumdioxid mit einer Stampfdichte von 120 g/l.

Ferner ist es vorteilhaft pyrogenes Siliziumdioxid mit einer BET-Oberfläche, bestimmt nach DIN 66131, von 50 bis 400 m²/g zu wählen. Besonders vorteilhaft ist eine BET-Oberfläche von 90-250 m²/g.

Das pyrogene Siliziumdioxid liegt bevorzugt zu 0,1 bis 10 Gew.-% in der erfindungsgemäßen Zubereitung vor.

Daneben kann die Zubereitung die üblichen in der Pharmazie und Kosmetik eingesetzten Hilfsstoffe enthalten, wie zum Beispiel Füllstoffe wie Kohlenhydrate, Zuckeralkohole, Stärke und Stärkederivate; Bindemittel, wie zum Beispiel Gelatine, Cellulose, Polyvinylpyrrolidon-Derivate; Sprengmittel, wie zum Beispiel Carboxymethylcellulose, Maisstärke und NatriumCarboxymethylstärke; Gleitmittel, wie zum Beispiel Talkum oder Polyethylenglykole; Schmier- und Formtrennmittel, wie zum Beispiel Magnesium- oder Calciumstearat oder Stearinsäure.

Verfahren zur Herstellung von pyrogenem Siliziumdioxid finden sich zum Beispiel in Ullmann's Encyclopedia of Industrial Chemistry, Vol. A23, Seite 635 ff., 5. Auflage, 1993.

US-6,217,909 beschreibt Zellulose-haltige Zusammensetzungen zur Herstellung von Tabletten mit einer verbesserten Kompressibilität. Die Zusammensetzungen enthalten auch Siliziumdioxid. Mehrere Marken von pyrogenem Siliziumdioxid werden erwährt (Aerosil OX50, Cab-O-Sil S-17, Cab-O-Sil EH-5). Es wird Siliziumdioxidpulver mit einer Schüttdichte von 20-100 g/l und mit einer spezifischen Oberfläche von 50-500 m² bevorzugt.

Pyrogenes Siliziumdioxid umfasst ebenfalls dotierte Oxide und Mischoxide bei denen der Siliziumdioxidgehalt mindestens 90% beträgt. Dotierte, pyrogene Siliziumdioxide können zum Beispiel erhalten werden nach dem in DE-A-196 50 500 beschriebenen Verfahren, wobei die Dotierung über ein Aerosol aus einer Salzlösung oder -suspension in eine Flamme, wie sie zur Herstellung pyrogener Oxide Verwendung findet, eingebracht wird. Ein Mischoxid mit einem Siliziumdioxidgehalt größer als 90 Gew.-% kann zum Beispiel nach dem in DE-A-199 19 635 beschriebenen Verfahren erhalten werden.

Das Siliziumdioxid kann ferner durch eine nachfolgende Behandlung mit einem Oberflächenmodifizierungsreagenz eine teilweise oder vollständig hydrophobierte Oberfläche erhalten. Verfahren hierzu finden sich zum Beispiel in DE-A-11 63 784, DE-A-196 16 781, DE-A-197 57 210 oder DE-A-44 02 370.

Das hochdisperse, pyrogene Siliziumdioxid erhält seine Stampfdichte entweder unmittelbar bei der Herstellung oder in einem nachgeschalteten Prozessschritt. So werden zum Beispiel in DE-A-32 38 427 und DE-A-37 41 846 Verdichtungsverfahren für das pyrogene Siliziumdioxid beschrieben. Besonders vorteilhaft für die erfindungsgemäße Zubereitung ist die in DE-A-37 41 846 angegebene Verdichtung mittels eines Vakuum-Drehfilters, der mit einem Preßband ausgestattet ist. Solche Produkte, die beispielsweise unter der Bezeichnung AEROSIL 200 VV vermarktet werden, zeichnen sich durch eine besondere einheitliche und homogene Struktur aus.

Für die Zubereitungen können auch Mischungen von pyrogenem Siliziumdioxid mit dotiertem Siliziumdioxid mit einem SiO₂-Anteil von 90%, mit Mischoxiden mit einem SiO₂-Anteil von 90% oder mehr und/oder hydrophobiertem Siliziumdioxid Verwendung finden.

Die Zubereitung kann hochdisperses, pyrogenes Siliciumdioxid mit einer Stampfdichte zwischen 80 und 250 g/l in Kombination mit jedem beliebigen pharmazeutischen Wirkstoff enthalten. Beispielhaft genannte pharmazeutische Wirkstoffe seien: α-Proteinase-Inhibitor, Abacavir, Abciximab, Acarbose, Acetylsalicylsäure, Acyclovir, Adenosin, Albuterol, Aldesleukin, Alendronat, Alfuzosin, Alosetron, Alprazolam, Alteplase, Ambroxol, Amifostin, Amiodaron, Amisulprid, Amlodipin, Amoxicillin, Amphetamin, Amphotericin, Ampicillin, Amprenavir, Anagrelid, Anastrozol, Ancrod, Anti-Hämophiliefaktor, Aprotinin, Atenolol, Atorvastatin, Atropin, Azelastin, Azithromycin, Azulen, Barnidipin, Beclomethason, Benazepril, Benserazid, Beraprost, Betamethason, Betaxolol, Bezafibrat, Bicalutamid, Bisabolol, Bisoprolol, Botulinus-Toxin, Brimonidin, Bromazepam, Bromocriptin, Budesonid, Bupivacain, Bupropion, Buspiron, Butorphanol, Cabergolin, Calcipotrien, Calcitonin, Calcitriol, Campher, Candesartan, Candesartan cilexetil, Captopril, Carbamazepin, Carbidopa, Carboplatin, Carvedilol, Cefaclor, Cefadroxil, Cefaxitin, Cefazolin, Cefdinir, Cefepim, Cefixim, Cefmetazol, Cefoperazon, Cefotiam, Cefoxopran, Cefoxitin, Cefpodoxim, Cefprozil, Ceftazidim, Ceftibuten, Ceftriaxon, Cefuroxim, Celecoxib, Celiprolol, Cephalexin, Cerivastatin, Cetirizin, Chloramophenicol, Cilastatin, Cilazapril, Cimetidin, Ciprofibrat, Ciprofloxacin, Cisaprid, Cisplatin, Citalopram, Clarithromycin, Clavulansäure, Clindamycin, Clomipramin, Clonazepam, Clonidin, Clopidogrel, Clotrimazol, Clozapin, Cromolyn, Cyclophosphamid, Cyclosporin, Cyproteron, Dalteparin, Deferoxamin, Desogestrel, Dextroamphetamin, Diazepam, Diclofenac, Didanosin, Digitoxin, Digoxin, Dihydroergotamin, Diltiazem, Diphtherie-Protein, Diphtherie-Toxoid, Divalproex, Dobutamin, Docetaxel, Dolasetron, Donepezil, Dornase-α, Dorzolamid, Doxazosin, Doxifluridin, Doxorubicin, Dydrogesteron, Ecabet, Efavirenz, Enalapril, Enoxaparin, Eperison, Epinastin, Epirubicin, Eptifibatid, Erythropoietin-α, Erythropoietin-β, Etanercept, Ethinylöstradiol, Etodolac, Etoposid, Faktor-VIII, Famciclovir, Famotidin, Faropenem, Felodipin, Fenofibrat, Fenoldopam, Fentanyl, Fexofenadin, Filgrastim, Finasterid, Flomoxef, Fluconazol, Fludarabin, Flunisolid, Flunitrazepam, Fluoxetin, Flutamid, Fluticason, Fluvastatin, Fluvoxamin, Follitropin-α, Follitropin-β, Formoterol, Fosinopril, Furosemid, Gabapentin, Gadodiamid, Ganciclovir, Gatifloxacin, Gemcitabin, Gestoden, Glatiramer, Glibenclamid, Glimepirid, Glipizid, Glyburid, Goserelin, Granisetron, Griseofulvin, Hepatitis-B-Antigen, Hyaluronasäure, Hycosin, Hydrochlorthiazid, Hydrocodon, Hydrocortison, Hydromorphon, Hydroxychloroquin, Hylan G-F 20, Ibuprofen, Ifosfamid, Imidapril, Imiglucerase, Imipenem, Immunoglobulin, Indinavir, Indomethacin, Infliximab, Insulin, Insulin, human, Insulin Lispro, Insulin aspart, Interferon-β, Interferon-α, Iod-125, Iodixanol, Iohexol, Iomeprol, Iopromid, Ioversol, Ioxoprolen, Ipratropium, Ipriflavon, Irbesartan, Irinotecan, Isosorbid, Isotretinoin, Isradipin, Itraconazol, Kaliumchlorazepat, Kaliumchlorid, Ketorolac, Ketotifen, Keuchhusten-Vakzin, Koagulationsfaktor-IX , Lamivudin, Lamotrigin, Lansoprazol, Latanoprost, Leflunomid, Lenograstim, Letrozol, Leuprolid, Levodopa, Levofloxacin, Levonorgestrel, Levothyroxin, Lidocain, Linezolid, Lisinopril, Lopamidol, Loracarbef, Loratadin, Lorazepam, Losartan, Lovastatin, Lysinacetylsalicylsäure, Manidipin, Mecobalamin, Medroxyprogesteron, Megestrol, Meloxicam, Menatetrenon, Meningokokken-Vakzin, Menotropin, Meropenem, Mesalamin, Metaxalon, Metformin, Methylphenidat, Methylprednisolon, Metoprolol, Midazolam, Milrinon, Minocyclin, Mirtazapin, Misoprostol, Mitoxantron, Moclobemid, Modafinil, Mometason, Montelukast, Morniflumat, Morphium, Moxifloxacin, Mykophenolat, Nabumeton, Nadroparin, Naproxen, Naratriptan, Nefazodon, Nelfinavir, Nevirapin, Niacin, Nicardipin, Nicergolin, Nifedipin, Nilutamid, Nilvadipin, Nimodipin, Nitroglycerin, Nizatidin, Norethindron, Norfloxacin, Octreotid, Olanzapin, Omeprazol, Ondansetron, Orlistat, Oseltamivir, Östradiol, Östrogene, Oxaliplatin, Oxaprozin, Oxolinsäure, Oxybutynin, Paclitaxel, Palivizumab, Pamidronat, Pancrelipase, Panipenem, Pantoprazol, Paracetamol, Paroxetin, Pentoxifyllin, Pergolid, Phenytoin, Pioglitazon, Piperacillin, Piroxicam, Pramipexol, Pravastatin, Prazosin, Probucol, Progesteron, Propafenon, Propofol, Propoxyphen, Prostaglandin, Quetiapin, Quinapril, Rabeprazol, Raloxifen, Ramipril, Ranitidin, Repaglinid, Reserpin, Ribavirin, Riluzol, Risperidon, Ritonavir, Rituximab, Rivastigmin, Rizatriptan, Rofecoxib, Ropinirol, Rosiglitazon, Salmeterol, Saquinavir, Sargramostim, Serrapeptase, Sertralin, Sevelamer, Sibutramin, Sildenafil, Simvastatin, Somatropin, Sotalol, Spironolacton, Stavudin, Sulbactam, Sulfaethidol, Sulfamethoxazol, Sulfasalazin, Sulpirid, Sumatriptan, Tacrolimus, Tamoxifen, Tamsulosin, Tazobactam, Teicoplanin, Temocapril, Temozolomid, Tenecteplase, Tenoxicam, Teprenon, Terazosin, Terbinafin, Terbutalin, Tetanus Toxoid , Tetrabenazin, Tetrazapam, Thymol, Tiagabin, Tibolon, Ticarcillin, Ticlopidin, Timolol, Tirofiban, Tizanidin, Tobramycin, Tocopherylnicotinat, Tolterodin, Topiramat, Topotecan, Torasemid, Tramadol, Trandolapril, Trastuzumab, Triamcinolon, Triazolam, Trimebutin, Trimethoprim, Troglitazon, Tropisetron, Tulobuterol, Unoproston, Urofollitropin, Valacyclovir, Valproinsäure, Valsartan, Vancomycin, Venlafaxin, Verapamil, Verteporfin, Vigabatrin, Vinorelbin, Vinpocetin, Voglibose, Warfarin, Zafirlukast, Zaleplon, Zanamivir, Zidovudin, Zolmitriptan, Zolpidem, Zopicion und deren Derivate. Unter pharmazeutischen Wirkstoffen sind jedoch auch andere Substanzen wie Vitamine, Provitamine, essentielle Fettsäuren, Extrakte pflanzlicher und tierischer Herkunft und Öle pflanzlicher und tierischer Herkunft zu verstehen.

Zu den pharmazeutischen Zusammensetzungen, in denen hochdisperses, pyrogenes Siliciumdioxid eingesetzt werden können, zählen auch pflanzliche Arzneizubereitungen und homöopathische Zubereitungen.

Bei den pharmazeutischen Zubereitungen kann es sich auch um sogenannte Retard- und Depotarzneiformen mit kontrollierter Wirkstofffreigabe handeln. Weiterhin können die erfindungsgemäßen pharmazeutischen Zubereitungen auch Teil therapeutischer Systeme wie etwa therapeutischer Systeme für die lokale Anwendung und transdermaler therapeutischer Systeme sein.

Gemäß einer vorteilhaften Ausführungsform enthalten die Zubereitungen als Wirkstoff Paracetamol, Acetylsalicylsäure oder Ibuprofen.

Bei den Zubereitungen kann es sich um beliebige feste, halbfeste oder flüssige Arzneiformen, bevorzugt für orale und/oder topische Anwendungen, handeln, z.B. in Suspensionen, Emulsionen, Aerosolen, Salben, Cremes, Gels, Pasten, Zäpfchen, Stiften, Pulvern, Pudern, Granulaten, Tabletten, Pastillen, Dragees, Filmtabletten, Hartgelatinekapseln, Weichgelatinekapseln, Extrudate, Mikrokapseln oder Mikrosphärulen. Besonders bevorzugt sind feste Arzneiformen wie z.B. Pulver, Puder, Granulate, Tabletten und Kapseln. Unter den Begriff "pharmazeutische Zusammensetzung" im Sinne der vorliegenden Erfindung fallen auch Vor- und Zwischenprodukte zur Herstellung von Granulaten, Tabletten, Kapseln, Suspensionen, Trockensäften und Trockentropfen. Solche Vor- und Zwischenprodukte können z.B. die Form eines Pulvers, Granulats oder Extrudats aufweisen.

Methoden zur Herstellung fester, halbfester und flüssiger Arzneiformen sind bekannt und werden in zahlreichen Veröffentlichungen und Lehrbüchern der pharmazeutischen Technologie beschrieben, vgl. zum Beispiel K.H. Bauer, K.-H. Frömming, C. Führer, Lehrbuch der pharmazeutischen Technologie, 6. Auflage, Wissenschaftliche Verlagsgesellschaft mbH Stuttgart 1999.

### Beispiele

Zubereitungen: Die pulverförmigen Einsatzstoffe werden in der angegebenen Reihenfolge auf 0,01 g genau eingewogen und in einer Glasflasche von Hand gemischt. Diese Mischung wird durch ein Sieb mit der Maschenweite 0,75 mm gesiebt und in einer Glasflasche mit einem Turbulamischer fünf Minuten homogenisiert.

**Tabelle 1: Zubereitungen (Angaben in Gew.-%)**

| | Zubereitung 1 | Zubereitung 2 |
|---|---|---|
| Paracetamol | 83,3 | - |
| Acetylsalicylsäure | - | 83,3 |
| Pulvercellulose | 13,3 | 10,4 |
| Maisstärke | 3,0 | 5,0 |
| Magnesiumstearat | 0,1 | - |
| Stearinsäure | - | 1,0 |
| Siliziumdioxid | 0,3 | 0,3 |

Ferner werden mit den Zubereitungen gemäß Tabelle 1 Tabletten gepresst und Kapseln gefüllt.

### Hartgelatine-Kapseln

Unter Verwendung eines Kapselfüllgeräts werden Hartgelatine-Kapseln der Größe 1 mit einem Leergewicht von 71 - 78 mg mit den Zubereitungen gemäß Tabelle 1 gefüllt. Es werden jeweils 60 Kapseln hergestellt und das mittlere Kapselgewicht bestimmt.

Die Werte für Zubereitung 1 befinden sich Tab. 2, für Zubereitung 2 in Tab. 3.

### Tabletten

Die Zubereitungen gemäß Tab. 1 werden bei gleichem Pressdruck mit einer Exzenterpresse zu Tabletten mit einem Gewicht von ca. 600 mg verpresst. Die Tablettenhärte wird mittels eines halbautomatischen Härtetesters an jeweils 10 Tabletten bestimmt.

Die Werte für Zubereitung 1 befinden sich Tab. 2, für Zubereitung 2 in Tab. 3.

**Tabelle 2: Eigenschaften der Zubereitung 1**

| Stampfdichte SiO₂ [g/l] | Fließnote^{*} | Tablettenhärte [N] | Kapselgewicht [mg] |
|---|---|---|---|
| 50 | gut bis befriedigend | 116 | 368 |
| 75 | gut bis befriedigend | 115 | 371 |
| 120 | sehr gut | 138 | 392 |
| 150 | sehr gut | 140 | 391 |

| | | | |
|---|---|---|---|
| *Fließnote bestimmt nach:Schriftenreihe Pigmente, Nummer 11 von Degussa, 6.Auflage | | | |

**Tabelle 3: Eigenschaften der Zubereitung 2**

| Stampfdichte SiO₂ [g/l] | Fließnote | Tablettenhärte [N] | Kapselgewicht [mg] |
|---|---|---|---|
| 50 | gut | 86 | 367 |
| 75 | gut | 91 | 365 |
| 120 | sehr gut | 130 | 380 |
| 150 | sehr gut | 134 | 385 |

Die Zubereitungen zeigen eindeutig Vorteile bei Fließverhalten, Tablettenhärte und Kapselgewicht.

## Patentansprüche

1. Verwendung von pyrogenem Siliziumdioxid als Hilfsstoff zur Herstellung von Kapseln oder Tabletten, **dadurch gekennzeichnet, dass** das pyrogene Siliziumdioxid eine Stampfdichte von 100 bis 200 g/l sowie eine Spezifische Oberfläche zwischen 90 und 250 m²/g aufweist.

2. Verwendung nach Anspruch 1, **dadurch gekennzeichnet, dass** der Anteil an pyrogenem Siliciumdioxid in der Kapsel oder Tablette zwischen 0,1 und 10 Gew.% liegt.

3. Verwendung nach den Ansprüchen 1 oder 2, **dadurch gekennzeichnet, dass** das pyrogene Siliziumdioxid ein Mischoxid mit einem Siliziumdioxidgehalt von 90 Gew.-% oder mehr, oder ein dotiertes Oxid mit einem Siliziumdioxidgehalt von 90 Gew.-% oder mehr umfasst.

4. Verwendung nach den Ansprüchen 1 bis 3, **dadurch gekennzeichnet, dass** die Kapseln oder Tabletten Paracetamol, Acetylsalicylsäure oder Ibuprofen enthalten.

## Claims

1. Use of pyrogenic silicon dioxide as an excipient in the manufacture of capsules or tablets, **characterized in that** the pyrogenic silicon dioxide has a tamped density ranging from 100 to 200 g/l and also a specific surface area between 90 and 250 m²/g.

2. Use according to claim 1, **characterized in that** the fraction of pyrogenic silicon dioxide in the capsule or tablet is between 0.1% and 10% by weight.

3. Use according to claim 1 or claim 2, **characterized in that** the pyrogenic silicon dioxide comprises a mixed oxide having a silicon dioxide content of 90% by weight or more or a doped oxide having a silicon dioxide content of 90% by weight or more.

4. Use according to claims 1 to 3, **characterized in that** the capsules or tablets comprise paracetamol, acetylsalicylic acid or ibuprofen.

## Revendications

1. Utilisation de silice pyrogène en tant qu'auxiliaire, pour la production de capsules ou de comprimés, **caractérisée en ce que** la silice pyrogène présente une densité tassée de 100 à 200 g/l ainsi qu'une surface spécifique comprise entre 90 et 250 m²/g.

2. Utilisation selon la revendication 1, **caractérisée en ce que** la proportion de silice pyrogène dans la capsule ou le comprimé est comprise entre 0,1 et 10 % en poids.

3. Utilisation selon les revendications 1 et 2, **caractérisée en ce que** la silice pyrogène comprend un oxyde mixte avec une teneur en silice de 90 % en poids ou plus ou un oxyde dopé avec une teneur en silice de 90 % en poids ou plus.

4. Utilisation selon les revendications 1 à 3, **caractérisée en ce que** les capsules ou les comprimés contiennent du paracétamol, de l'acide acétylsalicylique ou de l'ibuprofène.
